# EUROPEAN PATENT SPECIFICATION

(11) **EP 1 487 840 B1**
(45) Date of publication and mention of the grant of the patent: **13.06.2007**
(21) Application number: 03743335.6
(22) Date of filing: 26.02.2003
(51) Int. Cl.: C07D 491/22, A61K 31/4375, A61P 35/00

(54) **CRYSTALLINE POLYMORPHIC FORM OF IRINOTECAN HYDROCHLORIDE**
KRISTALLINE POLYMORPHE FORM VON IRINOTECAN-HYROCHLORID
FORME POLYMORPHIQUE CRISTALLINE DE CHLORHYDRATE D'IRINOTECAN

(30) Priority: 01.03.2002 US 360684 P
(43) Date of publication of application: 22.12.2004
(73) Proprietor: Pfizer Italia S.r.l., 04010 Latina (IT)
(72) Inventor: FORINO, Romualdo, I-20144 Milano (IT); BARBUGIAN, Natale, I-20158 Milano (IT); ZAMPIERI, Massimo, I-20052 Monza (Milano) (IT); TOMASI, Attilio, I-20147 Milano (IT)
(74) Representative: Modiano, Micaela Nadia
(86) International application number: PCT/EP2003/001948
(87) International publication number: WO 2003/074527

(56) References cited:
- SAWADA S ET AL: "SYNTHESIS AND ANTITUMOR ACTIVITY OF 20(S)-CAMPTOTHECIN DERIVATIVES:CARBAMATE-LINKED, WATER-SOLUBLE DERIVATIVES OF 7-ETHYL-10-HYDROXYCAMPTOTHECIN" CHEMICAL AND PHARMACEUTICAL BULLETIN, PHARMACEUTICAL SOCIETY OF JAPAN. TOKYO, JP, vol. 39, no. 6, 1 June 1991 (1991-06-01), pages 1446-1454, XP000653715 ISSN: 0009-2363 cited in the application
- HENEGAR K E ET AL: "PRACTICAL ASYMMETRIC SYNTHESIS OF (S)-4-ETHYL-7,8-DIHYDRO-4-HYDROXY-1 H-PYRANOL3,4-F INDOLIZINE-3,6,10(4H)-TRIONE, A KEY INTERMEDIATE FOR THE SYNTHESIS OF IRINOTECAN AND OTHER CAMPTOTHECIN ANALOGS" JOURNAL OF ORGANIC CHEMISTRY, AMERICAN CHEMICAL SOCIETY. EASTON, US, vol. 62, no. 19, 1997, pages 6588-6597, XP002935575 ISSN: 0022-3263 cited in the application

## Description

### SUMMARY OF THE INVENTION

This invention relates to a novel crystalline polymorphic form of the compound (*S*)-4,11-Diethyl-3,4,12,14-tetrahydro-4-hydroxy-3,14=dioxo-1 *H-*pyrano[3',4':6,7]-indolizino[1,2- *b*]quinolin-9-yl [1,4'-bipiperidine]-1'-carboxylate (irinotecan) hydrochloride. A process for preparing this novel polymorphic form, pharmaceutical compositions comprising it as an active ingredient and the use of the same and its pharmaceutical compositions for manufacturing a medicament for treating cancer is also within the scope of the present invention.

### BACKGROUND OF THE INVENTION

Irinotecan hydrochloride, the compound (*S*) -4,11-Diethyl-3,4,12,14-tetrahydro-4-hydroxy-3,14-dioxo-1 *H-*pyrano[3',4':6,7]-indolizino[1,2-*b*]quinolin-9-yl[1,4'-bipiperidine]-1'-carboxylate hydrochloride, having the formula: 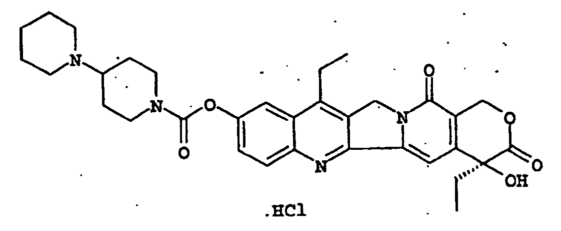 is a camptothecin analog and topoisomerase I inhibitor derived from a compound which occurs naturally in the Chinese tree, *Camptotheca acuminata*.

Since its approval in the United States in 1996, irinotecan hydrochloride trihydrate (CPT-11, CAMPTOSAR^{®}, injection, Pharmacia Corp.; Peapack, NJ) has undergone extensive clinical evaluation. In the past five years, the focus of development has evolved from evaluation of single-agent activity in refractory disease settings to evaluation of front-line irinotecan-based combination chemotherapy regimens and integration of irinotecan into combined modality regimens. Important studies have been performed clarifying the role of irinotecan treating colorectal and other gastrointestinal cancers, small cell and non-small cell lung cancer, and a variety of other malignancies.

CPT-11 has shown activity against a variety of tumor types, particularly refractory colorectal tumors, and it is used for the treatment of various forms of cancer. Its primary use is in the treatment of colon cancer, particularly advanced colon cancer. It is also of interest for treatment of other cancers, such as cancers of the lung, the stomach and the pancreas.

CPT-11 is usually administered in one of two treatment regimens. In one regimen, a dose of 125mg/m² of CPT-11 is given i.v. over a 90 minute period each week for four weeks. After a lapse of two weeks this is repeated, so that the patient receives CPT-11 in four weeks out of every six. In the other treatment regimen a dose of 350 mg/m² is given i.v. over 90 minutes, every third week. Thus, the one regimen operates with a six week cycle and the other regimen on a three week cycle.

CPT-11 is indicated as a component of first-line therapy in combination with 5-FU/LV for the treatment of patients with metastatic carcinoma of the colon or rectum. CPT-11 is also indicated for patients with metastatic carcinoma of the colon or rectum whose disease has recurred or progressed following initial 5-FU-based therapy. CPT-11 is the first topoisomerase I inhibitor with known activity in colorectal cancer, and the first FDA fully approved colorectal cancer treatment in over 40 years.

The antitumor activity of CPT-11 is attributed to an active metabolite, 7-ethyl-10-hydroxy 20 (S) camptothecin (SN-38), which is produced after enzymatic cleavage by carboxylesterases in the liver, small intestine and plasma. SN-38 is 100-fold more cytotoxic than CPT-11.

CPT-11 (CAMPTOSAR^{®}) is supplied as a sterile, pale yellow, clear, aqueous solution. It is available in two single-dose sizes: 2 ml-fill vials contain 40 mg irinotecan hydrochloride and 5 ml-fill vials contain 100 mg irinotecan hydrochloride. Each milliliter of solution contains 20 mg of irinotecan hydrochloride (on the basis of the trihydrate salt), 45 mg of sorbitol powder, and 0.9 mg of lactic acid. The pH of the solution has been adjusted to 3.5 (range, 3.0 to 3.8) with sodium hydroxide or hydrochloric acid. CAMPTOSAR^{®} is intended for dilution with 5% dextrose injection (D5W), or 0.9% sodium chloride injection, prior to intravenous infusion. The preferred diluent is 5% dextrose injection.

Yakult US patent No. 4,604,463 describes a broad family of camptothecin derivatives including irinotecan, its pharmaceutically acceptable salts and preparation thereof.

Pharmacia & Upjohn Co. US patent No. 6,121,451 discloses intermediates and processes for the synthesis of camptothecin derivatives, such as irinotecan hydrochloride.

Pharmacia and Upjohn S.p.A. International patent applications No. WO 01/10443 and WO 01/30351 describe oral pharmaceutical preparations comprising irinotecan hydrochloride.

Sawada et al., Chem. Pharm. Bull. Vol. 39, No. 6, 1446-54 (1991), describes the preparation of irinotecan from natural camptothecin in five chemical steps and 20% of overall yields. Sawada further discloses the crystal structure of irinotecan hydrochloride trihydrate (also known as CPT-11 that is the one that is currently used for the manufacturing of the commercially available product), as slightly pale yellow needles or crystalline powder by crystallization from water. The product is then dried (*in vacuo*) and equilibrated in a 75% relative humidity chamber for 70 hours. Crystalline form of irinotecan hydrochloride trihydrate as described by Sawada et al. is herein after referred to, for convenience, as "Form b".

A summary of the physical properties of Form b are reported below.

### Infrared absorbtion

IR (KBr) *v:* 1748 (lactone carbonyl), 1688 (carbamate carbonyl), 1663 (pyridone carbonyl), cm-1.

### Water solubility

Water solubility of Form b is at room temperature of about 10 mg/mL.

### Powder X-ray diffraction (PXRD)

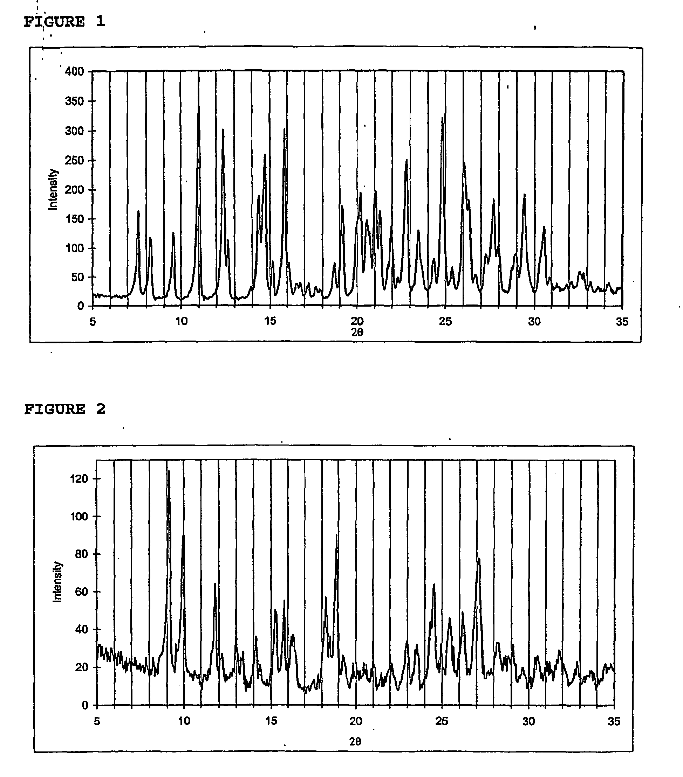

Form b was also characterized by its powder X-ray diffraction pattern, as shown in the spectrum of FIG. 1, comprising 2θ angle values of about 7.60; 8.30; 9.55; 11.00; and 12.40.

The Relative Intensity (%) of the mentioned characteristics reflection peaks of Form b at the 2θ angle values are reported in TABLE I.

**TABLE I**

| **Angle (°2θ)** | **Relative Intensity (%)** |
|---|---|
| 7.60 | 47.9 |
| 8.30 | 33.4 |
| 9.55 | 36.9 |
| 11.00 | 100.0 |
| 12.40 | 88.1 |

Form b was characterized with a principal reflection peak at 11.0 deg (2θ).

Polymorphism' is the property of some molecules to adopt more than one crystalline form in the solid state. A single molecule can give rise to a variety of solids having distinct physical properties that can be measured in a laboratory like its thermal behavior, e.g. melting point and differential scanning calorimetry ("DSC") thermogram, dissolution rate, flowability, X-ray diffraction pattern, infrared absorption spectrum and NMR spectrum. The differences in the physical properties of polymorphs result from the orientation and intermolecular interactions of adjacent molecules in the bulk solid. Accordingly, polymorphs are distinct solids sharing the same molecular formula which can yet have distinct advantageous and/or disadvantageous physical properties compared to other forms in the polymorph family. One property of a pharmaceutical compound that can vary depending upon its polymorphic form is its rate of dissolution in aqueous solvent. The rate of dissolution can have therapeutic consequences since it can affect the rate that an orally administered pharmaceutical is delivered to the bloodstream of a patient.

### SUMMARY OF THE INVENTION

It is an object of the invention to provide a novel polymorphic form of crystalline irinotecan hydrochloride, methods of use of the polymorph, and methods of manufacture of the polymorph. This and other objects of the invention are provided by one or more of the embodiments described below.

One embodiment of the invention provides a polymorphic form of crystalline irinotecan hydrochloride of formula: 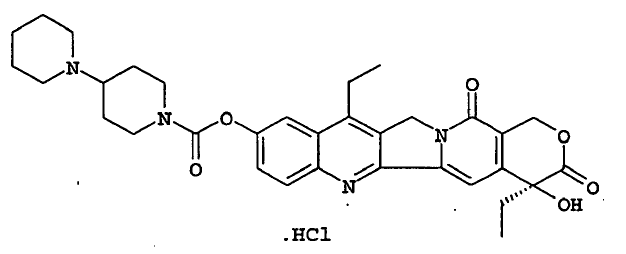 The polymorph is characterized by providing an X-ray powder diffraction pattern comprising 2θ angle values of about 9.15; 10.00; 11.80; 12.20; 13.00 and 13.40. The polymorph can further provide an infrared spectrum containing peaks at 1757, 1712 and 1667 cm-1. The polymorph can provide an X-ray powder diffraction pattern substantially in accordance with that shown in FIG. 2.

Another embodiment of the invention provides a process for preparing the polymorph. The method comprises stirring for a time ranging from about 2 to 48 hours a slurry of irinotecan hydrochloride as Form b or as amorphous, in acetonitrile or in acetone.

Still another embodiment of the invention provides a pharmaceutical composition that comprises a therapeutically effective amount of the polymorph as an active ingredient and a pharmaceutically acceptable excipient. The pharmaceutical composition can be suitable for injectable administration, oral administration and can be provided in an aqueous dosage form.

Yet another embodiment of the invention provides a method for the preparation of a pharmaceutical composition of irinotecan hydrochloride. The method comprises admixing a therapeutically effective amount of the polymorph of the invention with a pharmaceutically acceptable excipient.

Even another embodiment of the invention provides the use of the polymorph of the invention for manufacturing a medicament for treating a patient having a cancer. The treatment comprises administering a therapeutically effective amount of the polymorph. The cancer can be a gastrointestinal such as colorectal cancer.

Another embodiment of the invention provides a method for the preparation of an aqueous solution of irinotecan hydrochloride. The method comprises dissolving the polymorph of the invention into an aqueous solution at room temperature. The aqueous solution can have a pH value ranging from about 3.0 to about 3.8. The final concentration of irinotecan hydrochloride can be higher than about 10 mg/mL.

### BRIEF DESCRIPTION OF THE DRAWINGS

The invention is illustrated by reference to the accompanying drawings described below.
FIG.1 shows powder X-ray diffractograms of the Form b crystalline polymorph of irinotecan hydrochloride.
FIG. 2 shows powder X-ray diffractograms of the Form c crystalline polymorph of irinotecan hydrochloride.

### DETAILED DESCRIPTION OF THE INVENTION

It has now been found that irinotecan hydrochloride can exist in another crystalline polymorphic form. This novel crystalline form is fully characterized herein below and is referred to, for convenience, as "Form c".

Owing to its crystalline properties, the new Form c of irinotecan hydrochloride according to the invention has surprising advantages with regard to the crystalline Form b in terms of improved solubility performances.

Solubility enhancement has a significant advantage not only in terms of oral delivery of the active drug substance, but also in terms of improved manufacturability of the parenteral dosage form.

A more soluble and more rapidly dissolving crystal form of an active product ingredient has a significant biopharmaceutical advantage for the oral administration of drugs, being a more prompt dissolution rate behavior of the active drug substance coupled with an improved rate of absorption of the active drug per se through the gastrointestinal wall.

In particular, when solubility experimental tests comparing Form c and Form b have been carried out at strong acidic pH 1.2 (this pH value is representative of the environmental pH of the stomach, where the absorption processes through the gastrointestinal wall of acidic moieties starts), it was surprisingly noted that the Form c of irinotecan hydrochloride was 2 orders of magnitude more soluble with respect to the already known Form b (42 mg/mL vs 0.45 mg/mL).

Furthermore, in terms of technological advantages versus the manufacturing of parenteral dosage forms of irinotecan hydrochloride, the newly found Form c has the advantage of simplifying the current manufacturing procedure.

As stated above, CPT-11 (CAMPTOSAR^{®}) is supplied as a solution formulation where the active drug substance is 20 mg/mL concentrated in solution at a pH value of about 3.5. Being the equilibrium solubility of the Form b at that pH at room temperature lower than 20 mg/mL (its actual value, is in the order of magnitude of about 10 mg/mL), a heating process is needed during the manufacturing to completely solubilize the active at the required concentration. Such heating process generates stable association of Form b molecules in solution (as being known and described in the scientific literature as for example by Aiyama R. et al., Chemical Pharmaceutical Bulletin, 40 (10) 2810-2813), thus increasing also the solubility of the active drug substance at room temperature and allowing the manufacturing of physically stable ready to use 20 mg/mL solution formulations of Form b.

It has now been surprisingly found that the same formulation, with the same quality and long term physical stability, can be prepared with the newly synthesized Form c by simply dissolving the active drug substance in the selected diluent vehicle, thus avoiding any heating step. This fact allows a significant simplification of the manufacturing procedure of the final dosage form, both in terms of manufacturing time and manufacturing process itself. Manufacture the formulation at room temperature is likely dependent on the ability of the molecules of the new Form c to self associate in solution already at room temperature.

It is therefore another object of the present invention to use Form c for the preparation of an aqueous solution of irinotecan hydrochloride. In particular, the use of Form c is suitable for the preparation of an aqueous solution of irinotecan hydrochloride having a pH value ranging from about 3.0 to about 3.8. More particularly, Form c is suitable for the preparation of an aqueous solution having a pH value ranging from about 3.0 to 3.8 of irinotecan hydrochloride at a concentration higher than about 10 mg/mL.

In a further aspect, the present invention provides a method for the preparation of an aqueous solution of irinotecan hydrochloride, which comprises dissolving the required amount of Form c into an aqueous solution having a pH value ranging from about 3.0 to about 3.8 at room temperature.

No prior art of which applicants are aware describes Form c as now provided herein. To the best of applicants knowledge, Form c of the invention is previously unknown and are not suggested by the art.

It is therefore an object of the present invention to provide a new crystalline form of irinotecan hydrochloride, which is referred to as Form c.

One embodiment the present invention provides the Form c of crystalline irinotecan hydrochloride of formula: 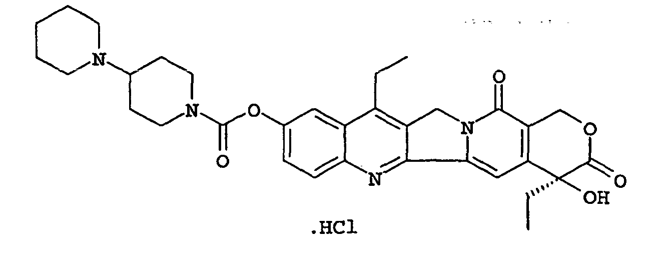 characterized by providing an X-ray powder diffraction pattern comprising 2θ angle values of about 9.15; about 10.00; about 11.80; about 12.20; about 13.00 and about 13.40. The relative intensity (%) of the characteristics reflection peaks of Form c at the 2θ angle values are reported in TABLE II.

**TABLE II**

| **Angle (°2θ)** | **Relative Intensity (%)** |
|---|---|
| 9.15 | 100.0 |
| 10.00 | 72.6 |
| 11.80 | 51.6 |
| 12.20 | 21.8 |
| 13.00 | 31.5 |
| 13.40 | 22.6 |

Form c was characterized with a principal reflection peak (100% of relative intensity) at 9.15 deg (2θ).

In particular, the Form c polymorph is characterized by an X-ray powder diffraction spectrum substantially in accordance with that shown in FIG. 2.

In a further embodiment, Form c is characterized by an infrared absorption spectrum comprising the following peaks (KBr) *v*: 1757(lactone carbonyl), 1712 (carbamate carbonyl) and 1667(pyridone carbonyl) cm-1.

Form b and Form c polymorphs of irinotecan hydrochloride can be readily distinguished by X-ray powder diffraction and infrared absorption spectra.

The relative intensities of the X-ray powder diffraction peaks can vary, depending upon the sample preparation technique, the sample mounting procedure and the particular instrument employed. Moreover, instrument variation and other factors can affect the 2θ values, therefore, the peak assignments can vary by plus or minus 0.2.

The regions of the diffractograms that are most useful in distinguishing the Form b and Form c polymorphs occurs in the region of about 7.00° and 14.00°, wherein all the mentioned characteristic peaks of the two forms are enclosed. For example, the Form c polymorph exhibits a strong peak at 9.15°, while the diffractogram of the Form b polymorph is substantially flat in this region, whilst Form b polymorph exhibits a strong peak at 11.00°, while the diffractogram of the Form c polymorph is substantially flat in this region.

Analysis by infrared (IR) is also an useful procedure for polymorphic characterization of crystalline irinotecan hydrochloride, which allows confirmation of the existence of the two polymorphic forms: Form b and Form c, through the detection of three different infra red absorption peaks, as displayed in the following Table III.

**TABLE III**

| | **Characteristic Infrared absorption peaks** | | |
|---|---|---|---|
| | **Lactone Carbonyl** group | **Carbamate Carbonyl** group | **Pyridone Carbonyl** group |
| **Form b** | 1747 | 1687 | 1662 |
| **Form c** | 1757 | 1712 | 1667 |

Water solubility of Form c is more than about 40mg/mL at room temperature.

The invention also provides a process for preparing the above Form c, which comprises stirring for a few hours, namely for a time ranging from about 2 to 48 hours, preferably from about 12 to 24 hours, a slurry of irinotecan hydrochloride (as Form b or as amorphous) in acetonitrile or in acetone. The starting materials for preparing Form c, can be obtained by a variety of procedures well known to those of ordinary skill in the art. For example, irinotecan hydrochloride as Form b or as amorphous can be prepared by the general procedure taught by Henegar K. E. et al., J. Org. Chem., 1997, 62, 6588-6597.

Owing to its crystalline properties, Form c of irinotecan hydrochlorid possesses greater solubility than the previously known form, which makes Form c not only more suitable for injectable dosage forms, but also for oral dosage forms with optimal delivery rate in the patient's bloodstream.

In fact the solubility of Form c versus Form b measured in the same experimental conditions (this means at room temperature at different pHs) is from 4 times (42 mg/mL vs 11 mg/mL' in water) to 100 times higher (42.5 mg/mL vs 0.45 mg/mL at pH 1.2). In particular, the solubility of the Form c is 5 times higher at room temperature in the buffering system (lactic acid buffer) currently used in the manufacturing process of the sterile injectable formulation known and marketed as CAMPTOSAR® INJECTION (54.4 mg/mL vs 10 mg/mL).

It is therefore a further object of the present invention to provide a pharmaceutical composition, which comprises a therapeutically effective amount of a polymorphic Form c of irinotecan hydrochloride as an active ingredient and a pharmaceutically acceptable excipient.

It is still another object of the present invention to provide a method for the preparation of a pharmaceutical composition of irinotecan hydrochloride, which comprises admixing a therapeutically effective amount of Form c with a pharmaceutically acceptable excipient.

Pharmaceutical compositions according to the invention can be prepared, for example, as parenteral, oral, transdermal, nasal or pulmonary dosage forms.

Compositions of the invention containing pharmaceutically acceptable excipients can be prepared by any of the well known techniques of pharmacy that comprise admixing the excipients with a drug or therapeutic agent.

For example, Form c may be formulated as an aqueous sterile solution for injectable administration purposes.

The pharmaceutical compositions according to the invention are useful for manufacturing a medicament for the prevention, amelioration, and/or treatment of benign and malignant tumors/neoplasias including cancer, such as, for example, brain cancer, bone cancer, epithelial cell-derived neoplasia (epithelial carcinoma) such as basal cell carcinoma, adenocarcinoma, esophageal cancer, small bowel cancer and stomach cancer, colon cancer, liver cancer, bladder cancer, pancreas cancer, ovary cancer, cervical cancer, lung cancers, breast cancer and skin cancer, prostate cancer, renal cell carcinoma, and other known cancers that effect epithelial cells throughout the body. Cancers for which compositions of the invention are contemplated to be particularly useful are gastrointestinal cancers, especially colorectal cancer, lung cancers, especially small cells lung cancer, cervical and pancreatic cancers.

Further disclosed is the use of Form C according to the invention for manufacturing a medicament for treating a patient having a cancer, especially a colorectal cancer. The treatment comprises administering a therapeutically effective amount of Form c according to the invention.

It is understood that the specific dose of a compound administered according to this invention to obtain a therapeutic effect will, of course, be determined by the particular circumstances surrounding the administration, including, for example, the age, weight, condition of the patient and administration route; specific dosage regimens can be fit to any particular subject on the basis of the individual need and the professional judgment of the person administering or supervising the administration of the aforesaid compounds.

The dosage range adopted will depend on the route of administration and on the age, weight and condition of the patient being treated. As an example, daily doses of the compounds of the invention, typically administered by parenteral route, for example, intravenously by bolus or infusion is from 1 to 1000 mg/m² body surface area, for instance from 10 to 500 mg/m². The dosages can be administered at once or can be divided into a number of smaller doses to be administered at varying intervals. A particular example of suitable schedule for parenteral administration of Form c is a 6-week dosing schedule of 125 mg/m² given i.v. over 90 minute of infusion on the first day of weeks 1-4. In another treatment regimen a dose of 350 mg/m² of Form c may be given i.v. over 90 minutes, every third week.

The term "treating" as used herein, unless otherwise indicated, means reversing, alleviating, ameliorating, inhibiting the progress of, or preventing the disorder or condition to which such term applies, or one or more symptoms of such disorder or condition. The term "treatment", as used herein, refers to the act of treating, as "treating" is defined immediately above.

### ANALYTICAL METHODS

### X-Ray Powder Diffraction

X-ray powder diffraction data were obtained with a Siemens D500. apparatus, irradiating powder samples with a CuKα graphite-monochromatic (40 kv 40 mA) source between 5° and 35° of 2θ angle at room temperature. The scan was made of 0.05° steps and the count time was 7 seconds per step.

### Infrared absorption

The infrared absorption spectra were obtained using on a Perkin-Elmer FT-IR PARAGON 1000 spectrometer at 4,0 cm-1 resolution. Data were digitised at 2 cm-1 intervals.

### EXAMPLES

### Example 1

### Preparation of irinotecan hydrochloride (Form c)

10.0 g of irinotecan·HCl·3H₂O Form b were suspended in 200 ml of acetone at room temperature.

The mixture was stirred for 24 hours at room temperature and then filtered. The product was dried in vacuo at 45°C for 18 hours and then placed in a chamber in presence of humidity for 24 hours. 9.4 g of Form c was obtained.

### Example 2

### Preparation of irinotecan hydrochloride (Form c)

10.0 g of irinotecan·HCl·3H₂O Form b were suspended in 150 ml of acetonitrile at room temperature.

The mixture was stirred for 48 hours at room temperature and then filtered. The product was dried in vacuo at 45°C for 18 hours and then placed in a chamber in presence of humidity for 4 hours. 9.1 g of Form c was obtained.

### Example 3

### Preparation of irinotecan hydrochloride (Form c)

10.0 g of irinotecan·HCl amorphous were suspended in 200 ml of acetone at room temperature.

The mixture was stirred for 24 hours at room temperature and then filtered. The product was dried in vacuo at 45°C for 18 hours and then placed in a chamber in presence of humidity for 3 hours. 10.0 g of Form c was obtained.

### Example 4

### Preparation of irinotecan hydrochloride (Form c)

10.0 g of irinotecan·HCl amorphous were suspended in 250 ml of acetonitrile at room temperature.

The mixture was stirred for 48 hours at room temperature and then filtered. The product was dried *in vacuo* at 45°C for 24 hours and then placed in a chamber in presence of humidity for 5 hours. 8.8 g of Form c was obtained.

### Example 5

### Solubility determination of irinotecan hydrochloride Form c in comparison with Form b

Solubility determination of irinotecan hydrochloride Form c in comparison with Form b has been carried out in the following dissolution media: a) deionized water; b) pH 1.2 buffer (2 g NaCl + 7 mL HCl 37% w/w to 1 Liter); c) Lactate buffer pH 3.5 (this is the formulation currently used for the manufacturing of the parenteral dosage form; 45 mg/ml D-Sorbitol 0.9 mg/ml Lactic Acid in water at pH 3.5. The pH is adjusted to the final value with NaOH 1N). An excess solid was put in glass flasks in the presence of the appropriate dissolution media and the suspensions were shaken at room temperature for 24 hours. Samples were withdrawn after 15, 30, 45, 60 min and 24 hours, filtered and analyzed by means of HPLC assay.

The results are summarized in the following Table IV.

**TABLE IV**

| **Time** | **Irinotecan hydrocholoride concentration (mg/ml)** | | | | | |
|---|---|---|---|---|---|---|
| | **Form b** | | | **Form c** | | |
| | **a** | **b** | **c** | **a** | **b** | **c** |
| **15 MIN** | 11.03 | 0.45 | 10.27 | 42.04 | 42.53 | 54.45 |
| **30 MIN** | 12.10 | 0.42 | 10.38 | 41.50 | 42.05 | 55.23 |
| **60 MIN** | 12.12 | 0.44 | 10.17 | 40.62 | 40.68 | 53.81 |
| **120 MIN** | 11.80 | 0.40 | 11.13 | 41.15 | 37.95 | 54.80 |
| **24 HOURS** | 11.15 | 0.26 | 11.06 | 35.05 | 0.70 | 41.95 |

| | | | | | | |
|---|---|---|---|---|---|---|
| a = deionized water; b = pH 1.2 buffer; c = lactic acid pH 3.5 | | | | | | |

The above-tabulated data provide evidence of the improved solubility of Form c when compared with the known Form b.

### Example 6

### An Injectable Solution of Form c comprises, for example, Injectable solution of Form c

1 ml of injectable solution contains:
20 mg of Form c (as salt equivalent);
45 mg of sorbitol powder; and
0.9 mg of lactic acid.

The pH of the solution is adjusted to 3.5 (range, 3.0 to 3.8) with sodium hydroxide or hydrochloric acid.

An injectable solution is available in, for example, single-dose amber glass vials in the following package sizes: 2 mL or 5 mL. This is packaged in a backing/plastic blister to protect against inadvertent breakage and leakage.

The injectable solution of Form c is intended for dilution with 5% dextrose injection, or 0.9% sodium chloride injection, prior to intravenous infusion. The preferred diluent is 5% dextrose injection. All the manufacturing procedure have been carried on at room temperature.

## Claims

1. A polymorphic form of crystalline irinotecan hydrochloride of formula: 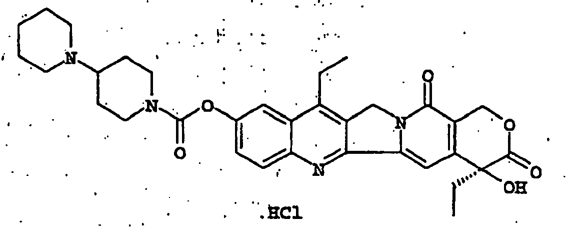 **characterized by** providing an X-ray powder diffraction pattern comprising 2θ angle values of 9.15; 10.00; 11.80; 12.20; 13.00 and 13.40.

2. The polymorph of claim 1, which provides an infrared spectrum containing peaks at 1757, 1712 and 1667 cm-1.

3. The polymorph of claim 1 which provides an X-ray powder diffraction pattern substantially in accordance with that shown in FIG. 2.

4. The polymorph of claim 2 which provides an X-ray powder diffraction pattern substantially in accordance with that shown in FIG. 2.

5. A process for preparing the polymorph of claim 1, which comprises stirring for a time ranging from 2 to 48 hours a slurry of irinotecan hydrochloride as Form b **characterized by** its powder X-ray diffraction pattern comprising 2θ angle values of 7.60; 8.30; 9.55; 11.00; and 12.40, or as amorphous, in acetonitrile or in acetone.

6. A pharmaceutical composition that comprises a therapeutically effective amount of the polymorph of claim 1 as an active ingredient and a pharmaceutically acceptable excipient.

7. The composition of claim 6, wherein the composition is suitable for injectable administration.

8. The composition of claim 6, wherein the composition is suitable for oral administration.

9. The composition of claim 6, wherein the composition is in an aqueous dosage form.

10. A method for the preparation of a pharmaceutical composition of irinotecan hydrochloride, which comprises admixing a therapeutically effective amount of the polymorph of claim 1 with a pharmaceutically acceptable excipient.

11. Use of polymorph of claim 1 for manufacturing a medicament for treating a patient having cancer by administration of a therapeutically effective amount.

12. The use of claim 11, wherein the cancer is a gastrointestinal cancer.

13. The use of claim 12, wherein the gastrointestinal cancer is a colorectal cancer.

14. Use of a pharmaceutical composition of claim 6 for manufacturing a medicament for treating a patient having a cancer.

15. The use of claim 14, wherein the cancer is a gastrointestinal cancer.

16. The use of claim 15, wherein the gastrointestinal cancer is a colorectal cancer.

17. A method for the preparation of an aqueous solution of irinotecan hydrochloride, that comprises dissolving the polymorph of claim 1 into an aqueous solution at room temperature.

18. The method of claim 17, wherein the aqueous solution has a pH value ranging from 3.0 to 3.8.

19. The method of claim 18, wherein the final concentration of irinotecan hydrochloride is higher than 10 mg/mL.

## Patentansprüche

1. Eine polymorphe Form von kristallinem Irinotecanhydrochlorid der Formel: 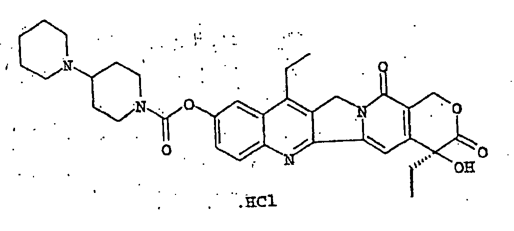 **gekennzeichnet durch** Bereitstellung eines Röntgenpulverbeugungs-Diagramms, das 2 θ Winkelwerte von 9,15; 10,00; 11,80; 12,20; 13, 00; und 13,40 umfasst.

2. Das Polymorph von Anspruch 1, welches ein Infrarotspektrum, das Peaks bei 1757, 1712 und 1667 cm⁻¹ enthält, bereitstellt.

3. Das Polymorph von Anspruch 1, welches ein Röntgenpulverbeugungs-Diagramm im wesentlichen übereinstimmend mit dem, das in Figur 2 gezeigt ist, bereitstellt.

4. Das Polymorph von Anspruch 2, welches ein Röntgenpulverbeugungs-Diagramm im wesentlichen übereinstimmend mit dem, das in Figur 2 gezeigt ist, bereitstellt.

5. Ein Verfahren zur Herstellung des Polymorphs von Anspruch 1, welches Rühren einer Aufschlämmung von Irinotecanhydrochlorid als Form b, **gekennzeichnet durch** sein Röntgenpulverbeugungs-Diagramm, das 2 θ Winkelwerte von 7,60; 8,30; 9,55; 11,00; und 12,40 umfasst, oder als Amorph, in Acetonitril oder in Aceton für einen Zeitraum im Bereich von 2 bis 48 Stunden, umfasst.

6. Eine pharmazeutische Zusammensetzung, die eine therapeutisch wirksame Menge des Polymorphs von Anspruch 1 als einen aktiven Inhaltsstoff und ein pharmazeutisch verträgliches Bindemittel umfasst.

7. Die Zusammensetzung von Anspruch 6, worin die Zusammensetzung für eine injizierbare Verabreichung geeignet ist.

8. Die Zusammensetzung von Anspruch 6, worin die Zusammensetzung für die orale Verabreichung geeignet ist.

9. Die Zusammensetzung von Anspruch 6, worin die Zusammensetzung in einer wässerigen Dosierform vorliegt.

10. Ein Verfahren für die Herstellung einer pharmazeutischen Zusammensetzung von Irinotecanhydrochlorid, welches das Mischen einer therapeutisch wirksamen Menge des Polymorphs von Anspruch 1 mit einem pharmazeutisch verträglichen Bindemittel umfasst.

11. Verwendung des Polymorphs von Anspruch 1 zur Herstellung eines Medikaments zur Behandlung eines Patienten, der Krebs hat, durch Verabreichung einer therapeutisch wirksamen Menge.

12. Die Verwendung von Anspruch 11, worin der Krebs ein gastrointestinaler Krebs ist.

13. Die Verwendung von Anspruch 12, worin der gastrointestinale Krebs ein Colorektal-Krebs ist.

14. Verwendung einer pharmazeutischen Zusammensetzung von Anspruch 6, zur Herstellung eines Medikaments zur Behandlung eines Patienten, der Krebs hat.

15. Die Verwendung von Anspruch 14, worin der Krebs ein gastrointestinaler Krebs ist.

16. Die Verwendung von Anspruch 15, worin der gastrointestinale Krebs ein Colorektal-Krebs ist.

17. Ein Verfahren für die Herstellung einer wässerigen Lösung von Irinotecanhydrochlorid, das das Auflösen des Polymorphs von Anspruch 1 in einer wässerigen Lösung bei Raumtemperatur umfasst.

18. Das Verfahren von Anspruch 17, worin die wässerige Lösung einen pH-Wert im Bereich von 3,0 bis 3,8 hat.

19. Das Verfahren von Anspruch 18, worin die Endkonzentration von Irinotecanhydrochlorid höher als 10 mg/ml ist.

## Revendications

1. Forme polymorphe de chlorhydrate d'irinotécan cristallin, de formule : 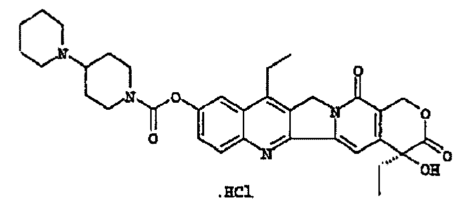 **caractérisée en ce qu'**elle présente un diagramme de diffraction des rayons X sur poudre comprenant des valeurs d'angle 2θ de 9,15 ; 10,00 ; 11,80 ; 12,12 ; 13,00 et 13,40.

2. Forme polymorphe suivant la revendication 1, qui présente un spectre infrarouge contenant des pics à 1757, 1712 et 1667 cm⁻¹.

3. Forme polymorphe suivant la revendication 1, qui présente un diagramme de diffraction des rayons X sur poudre correspondant substantiellement à celui représenté sur la figure 2.

4. Forme polymorphe suivant la revendication 2, qui présente un diagramme de diffraction des rayons X sur poudre correspondant substantiellement à celui représenté sur la figure 2.

5. Procédé pour la préparation de la forme polymorphe de la revendication 1, qui comprend l'agitation pendant un temps allant de 2 à 48 heures d'une suspension de chlorhydrate d'irinotécan sous la Forme b, **caractérisée par** son diagramme de diffraction des rayons X sur poudre comprenant des valeurs d'angle 2θ de 7,60 ; 8,30 ; 9,55 ; 11,00 ; et 12,40, ou sous la forme amorphe, dans de l'acétonitrile ou dans de l'acétone.

6. Composition pharmaceutique qui comprend une quantité thérapeutiquement efficace de la forme polymorphe de la revendication 1 comme ingrédient actif et un excipient pharmaceutiquement acceptable.

7. Composition suivant la revendication 6, la composition étant apte à l'administration par injection.

8. Composition suivant la revendication 6, la composition étant apte à l'administration orale.

9. Composition suivant la revendication 6, la composition étant sous une forme posologique aqueuse.

10. Procédé pour la préparation d'une composition pharmaceutique de chlorhydrate d'irinotécan, qui consiste à mélanger une quantité thérapeutiquement efficace de la forme polymorphe de la revendication 1 avec un excipient pharmaceutiquement acceptable.

11. Utilisation de la forme polymorphe de la revendication 1 pour la production d'un médicament destiné au traitement d'un patient présentant un cancer, par administration d'une quantité thérapeutiquement efficace.

12. Utilisation suivant la revendication 11, dans laquelle le cancer est un cancer gastro-intestinal.

13. Composition suivant la revendication 12, dans laquelle le cancer gastro-intestinal est un cancer colorectal.

14. Utilisation d'une composition pharmaceutique de la revendication 6 pour la production d'un médicament destiné au traitement d'un patient présentant un cancer.

15. Utilisation suivant la revendication 14, dans laquelle le cancer est un cancer gastro-intestinal.

16. Utilisation suivant la revendication 15, dans laquelle le cancer gastro-intestinal est un cancer colorectal.

17. Procédé pour la préparation d'une solution aqueuse de chlorhydrate d'irinotécan, qui comprend la dissolution de la forme polymorphe de la revendication 1 dans une solution aqueuse à température ambiante.

18. Procédé suivant la revendication 17, dans laquelle la solution aqueuse a un pH compris entre 3,0 et 3,8.

19. Procédé suivant la revendication 18, dans lequel la concentration finale de chlorhydrate d'irinotécan est supérieure à 10 mg/ml.
